# EUROPÄISCHE PATENTSCHRIFT

(11) **EP 1 072 590 B1**
(45) Veröffentlichungstag und Bekanntmachung des Hinweises auf die Patenterteilung: **13.04.2005**
(21) Anmeldenummer: 00114930.1
(22) Anmeldetag: 18.07.2000
(51) Int. Cl.: C07C 319/26, C07C 321/04, C07C 321/14, C07C 323/52, C10M 135/02, C10M 135/04, C10M 135/06

(54) **Verfahren zur Verhinderung der Schwefelwasserstoff- und/oder Mercaptanemission aus geschwefelten organischen Verbindungen**
Process for the prevention of hydrogen sulphide and/or mercaptan emission from sulphurised organic compounds
Procédé d'empêchement d'émission de sulfure d'hydrogène et/ou des mércaptanes des composés organiques sulfurés

(30) Priorität: 29.07.1999 DE 19935672
(43) Veröffentlichungstag der Anmeldung: 31.01.2001
(73) Patentinhaber: Rhein Chemie Rheinau GmbH, 68219 Mannheim (DE)
(72) Erfinder: Heiliger, Ludger, Dr., 67433 Neustadt/Weinstrasse (DE); Pauli, Alfred, Dr., 68799 Reilingen (DE); Hegmann, Joachim, Dr., 67117 Limburgerhof (DE); Wühr, Michael, 69493 Hirschberg (DE); Fessenbecker, Achim, Dr., 68753 Waghäusel-Kirrlach (DE); Schilling, Kurt, 68723 Schwetzingen (DE)
(74) Vertreter: Pettrich, Klaus-Günter, Dr.

(56) Entgegenhaltungen:
- EP-A- 0 531 842
- WO-A-90/02787
- US-A- 4 070 295
- US-A- 4 778 609
- US-A- 4 904 402
- US-A- 4 994 090
- US-A- 5 028 340
- US-A- 5 062 976

## Beschreibung

Die vorliegende Erfindung betrifft ein Verfahren zur Verhinderung der Schwefelwasserstoff- und/oder Mercaptanemission aus geschwefelten organischen Verbindungen, welche üblicherweise einen hohen Schwefelgehalt aufweisen.

Es ist bekannt, daß schwefelhaltige organische Verbindungen, die einen hohen Gehalt an Schwefel aufweisen und als sogenannte "Schwefelträger" dienen, bei deren Einsatz z.B. als (EP-Additive) in Schmierstoffe leicht zur Emission von Schwefelwasserstoff- und/oder Mercaptanen neigen, insbesondere wenn diese schwefelhaltigen Schmierstoffe erhöhten Temperaturen und Belastungen bei deren Einsatz ausgesetzt sind.

Eine von vielen Anforderungen an solchen schwefelhaltigen Verbindungen ist, daß möglichst keine Emission von Schwefelwasserstoff- und/oder Mercaptanen auftritt, da diese Verbindungen nicht nur geruchsbelästigend sind, sondern auch als gesundheitlich bedenklich anzusehen sind.

Es hat daher nicht an Versuchen gefehlt, die Emission von Schwefelwasserstoffund/oder Mercaptanen bei schwefelhaltigen organischen Verbindungen mit einem hohen Schwefelgehalt zu unterbinden, durch entsprechende Zusätze zu den schwefelhaltigen organischen Verbindungen oder durch Behandlung der schwefelhaltigen organischen Verbindungen mit entsprechenden Verbindungen, die eine solche Emission zu verhindern vermögen.

Beispielsweise wird in US 4,778,609 beschrieben, daß sterisch gehinderte Imine in der Lage sind die Schwefelwasserstoff-Freisetzung bei schwefelhaltigen Schmierstoffen zu verhindern. Nachteilig dabei ist, daß die eingesetzten Imine als ein (Form)aldehyddepot zu betrachten sind, so daß mit einer Freisetzung von (Form)-aldehyd gerechnet werden muß, was unter gesundheitlichen und ökologischen Gesichtspunkten als nachteilig anzusehen ist.

In US 4,873,006 wird eine Methode beschrieben, die die Freisetzung von Schwefelwasserstoff verhindern soll aus organischen Verbindungen mit aktivem Schwefelgehalt, in dem diese schwefelhaltigen Verbindungen mit den Reaktionsprodukten aus langkettig substituierten Polycarbonsäuren mit Hydroxylalkyl(poly)aminen, gegebenenfalls in Gegenwart von Alkali- oder Erdalkalimetallen enthaltenden Verbindungen, behandelt werden. Nachteilig bei dieser Methode ist vor allem der Einsatz von solchen metallhaltigen Verbindungen, was heutzutage aus ökologischen Gründen unerwünscht ist. Außerdem sind die zur Behandlung der schwefelhaltigen Verbindungen eingesetzten Reaktionsprodukte aus Polycarbonsäuren und Hydroxylaminen kompliziert herzustellen bzw. aufgebaut, was das Verfahren weniger wirtschaftlich macht.

Weiterhin ist bekannt, aus Ullmanns's Encyclopedia of Industrial Chemistry, Vol. A26, Page 775, Fifth Completely Revised Edition 1995, VCH Verlagsgesellschaft, ISBN 3-527-20126-2 sowie Houben-Weyl, Methoden der organischen Chemie, Band E11/Teil 1 Seite 136 ff, Georg Thieme Verlag Stuttgart, 1985, Schwefelwasserstoff und Mercaptane zu oxidieren und damit unschädlich zu machen durch deren Umsetzung mit Wasserstoffperoxid. Prinzipieller Nachteil dieser Methode ist, daß es sich dabei um eine Zweiphasenreaktion handelt, die einen erhöhten technischen Aufwand erfordert, was diese Methode weniger wirtschaftlich macht. Weiterhin ist es nicht möglich, mit Wasserstoffperoxide alle schwefelhaltigen, organischen Verbindungen zu oxidieren, da sich einige Mercaptane, z.B. auf Basis von α-Olefinen, auf diese Weise nicht oder nur sehr unvollständig oxidieren lassen. Zusätzlich können durch Überoxidation sehr leicht unerwünschte Nebenprodukte entstehen, die sich negativ auf das Löslichkeitsverhalten des organischen Schwefelträgers auswirken. Darüber hinaus wirkt sich die Wasserstoffperoxid-Behandlung, insbesondere dann, wenn sie mehrmals wiederholt werden muß, qualitätsverschlechternd, z.B. farbvertiefend auf die Schwefelträger aus.

Aufgabe der vorliegenden Erfindung ist es nun, ein einfaches, möglichst für alle Arten von organischen Schwefelträgern anwendbares Verfahren zur Verhinderung der Schwefelwasserstoff- und/oder Mercaptan-Emission zur Verfügung zu stellen, das ohne den Einsatz zusätzlicher Metallverbindungen, ohne Freisetzung von (Form)aldehyd und mit einem Minimum an unerwünschten Nebenreaktionen auskommt.

Es wurde nun gefunden, daß die Verwendung von organischen Hydroperoxiden die Stabilisierung von organischen, schwefelhaltigen Verbindungen mit einem hohen Schwefelgehalt bewerkstelligen kann, unabhängig davon auf welcher Rohstoffbasis (synthetischer oder nativer) diese schwefelhaltigen Verbindungen hergestellt wurden.

Gegenstand der vorliegenden Erfindung ist daher ein Verfahren zur Verhinderung der Schwefelwasserstoff- und/oder Mercaptan-Emission aus geschwefelten organischen Verbindungen mit hohem Schwefelgehalt, das dadurch gekennzeichnet ist, daß man die geschwefelten organischen Verbindungen mit 0,01 bis 10 Gew.-% eines organischen Hydroperoxids behandelt.

Bevorzugt werden die geschwefelten organischen Verbindungen mit 0,05 bis 5, insbesondere 0,1 bis 1,0 Gew.-%, bezogen auf das gesamte Reaktionsgemisch, eines organischen Hydroperoxids, behandelt.

Als organische Hydroperoxide, die für das erfindungsgemäße Verfahren eingesetzt werden können, kommen beispielsweise in Frage tert.-Butylhydroperoxid, tert.-Amylhydroperoxid, Cumolhydroperoxid sowie Diisopropylbenzolmonohydroperoxid. Selbstverständlich ist es auch möglich, andere als die genannten organischen Hydroperoxide einzusetzen. Bevorzugt wird Cumolhydroperoxid eingesetzt, insbesondere in Form seiner 10 bis 90 gew.-%igen Lösung, insbesonders seiner 70 bis 80 gew.-%igen Lösung in Cumol. Selbstverständlich können die anderen organischen Hydroperoxide ebenfalls in Lösung eingesetzt werden. Geeignet sind hierfür alle inerten, organischen Lösungsmittel.

Die Behandlung der geschwefelten organischen Verbindungen mit den organischen Hydroperoxiden, die sowohl einzeln als auch im Gemisch untereinander eingesetzt werden können, geschieht üblicherweise bei Reaktionstemperaturen von ca. 0 bis 150°C, bevorzugt 30 bis 120°C, insbesondere 50 bis 80°C, wobei die Zersetzungstemperatur der einzusetzenden Hydroperoxide die Temperatur nach oben hin begrenzt. Die Behandlungszeit der geschwefelten organischen Verbindungen mit den Hydroperoxiden hängt u.a. von dem jeweilig eingesetzten Hydroperoxid ab und beträgt ca. 1 bis 120 Minuten, vorzugsweise 5 bis 60 Minuten, insbesondere 15 bis 45 Minuten.

Als Hydroperoxide eignen sich insbesondere die oben beispielhaft erwähnten, das sind solche, die sich in der zu behandelnden geschwefelten organischen Verbindungen lösen und deren Lagerung und Handhabung ohne allzu großen technischen Aufwand möglich ist.

Werden die organischen Hydroperoxide in Lösung eingesetzt, so kann nach Bedarf das organische Lösungsmittel, beispielsweise das Cumol, bei Einsatz von Cumolhydroperoxid, nach beendeter Reaktion durch Vakuumdestillation entfernt werden.

Selbstverständlich ist es möglich, den organischen Hydroperoxiden noch andere aus dem Stand der Technk bekannten Verbindungen zuzusetzen, die die Emission von Schwefelwasserstoff und/oder Mercaptanen zu unterbinden vermögen. Beispielsweise Wasserstoffperoxid, sterisch gehinderte Imine oder die Umsetzungsprodukte aus langkettig substituierten Polycarbonsäuren mit Hydroxylalkyl(poly)aminen, gegebenenfalls in Gegenwart von Alkali- oder Erdalkalimetallen enthaltenden Verbindungen.

Mit dem erfindungsgemäßen Verfahren können alle schwefelhaltigen organischen Verbindungen mit einem hohen Schwefelgehalt, üblicherweise über 10 Gew.-%, behandelt werden, die beispielsweise in Schmierstoffen als EP-Additive dienen. Solche schwefelhaltigen organischen Verbindungen werden in bekannter Weise hergestellt durch Schwefelung ungesättigter organischer Verbindungen, wobei polysulfidische Verbindungen erhalten werden mit mehreren Schwefelatomen in der Kette.

Als ungesättigte organischer Verbindungen kommen solche in Frage mit einer oder mehrer Doppelbindungen. Die ungesättigten organischen Verbindungen können verzweigte oder unverzweigte aliphatische, cycloaliphatische oder aromatische Kohlenwasserstoffe mit 2 bis 50 C-Atomen sein. Beispiele für solche ungesättigten organischen Verbindungen sind Isobutylen, Propylen sowie deren entsprechende Di-, Tri- oder Tetrameren, Polyisobutylen, 1-Octen, 1-Decen, 1-Dodecen, C₂₀-C₂₄-Poly-α-olefine, Cyclohexene, Dicyclopentadien sowie Octadecenol.

Darüber hinaus kommen als ungesättigter organische Verbindungen auch natürlich Rohstoffe in Frage, wie tierische oder pflanzliche Fette und Öle. Sie enthalten Gemische von Mono-, Di-, Triglyceriden von gesättigten und ungesättigten Fettsäuren. Auch ungesättigte Wachsester hören dazu. Beispiele für solche Rohstoffe sind Sonnenblumenöl, Rüböl, Sojaöl, Erdnußöl, Rizinusöl, Lardöl, Talkfett sowie Fischöl.. Selbstverständlich können die genannten Fette und Öle auch in Form ihrer entsprechenden Ester eingesetzt werden.

Die Schwefelung der ungesättigten organischen Verbindungen ist bekannt und wird üblicherweise mit Schwefel und/oder Schwefelwasserstoff mit oder ohne Katalysator unter Normaldruck oder Überdruck bei erhöhter Temperatur durchgeführt. Nach der Schwefelung der eingesetzten organischen Verbindungen wird das Reaktionsgemisch zur Entfernung leicht-flüchtiger Verbindungen mit Stickstoff ausgeblasen, gegebenenfalls kann daran eine Vakuumbehandlung in Form einer Destillation oder Aufarbeitung in Dünnschichtverdampfer erfolgen. Dies geschieht im allgemeinen bei Temperaturen von eta 50 bis 150°C und einem Druck von ca. 5 bis 500 mbar.

Im allgemeinen reicht die beschriebene Nachbehandlung der geschwefelten organischen Produkte jedoch nicht aus, um geschwefelte Produkte herzustellen, bei denen keine Schwefelwasserstoff- und/oder Mercaptan-Emission mehr möglich ist.

Nach dem erfindungsgemäßen Verfahren gelingt es jedoch, geschefelte organische Verbindungen mit einem hohen Schwefelgehalt zu erhalten, die praktisch keine Emission an Schwefelwasserstoff und/oder Mercaptanen mehr aufweisen, wenn sie beispielsweise in Schmierstoffen als EP-Additive unter erhöhter Belastung (Temperatur, Druck) eingesetzt werden.

### Beispiele

Um die unwünschten Emissionen an Schwefelwasserstoff und/oder Mercaptanen bestimmen zu können, eignet sich der nachstehend beschriebene sogenannte Schwefelwasserstoff-Test. Dieser wird wie folgt ausgeführt:

15 g der zu untersuchenden Substanz werden in eine 100 ml-Weithalsflasche eingewogen. Ein Streifen Bleiacetatpapier wird mit Wasser angefeuchtet (das erhöht die Empfindlichkeit des Tests) und so geknickt, daß er zwischen Deckel und Glasgewinde der Flasche geklemmt werden kann. Ca 2/3 des Streifens müssen frei in der Flasche im Gasraum über der zu untersuchenden Substanz hängen. Das Bleiacetatpapier darf nicht mit der Substanz in Berührung kommen. Die Flasche wird fest verschlossen und für 30 Minuten im Trockenschrank bei 100°C aufbewahrt. Nach dem Abkühlen wird die Verfärbung des Bleiacetatpapieres ausgewertet. (Durch H₂S färbt es sich schwarz, durch Mercaptane braun). Die Auswertung erfolgt in einer vierstufigen Skala:
Note 1: keine Verfärbung
Note 2: leichte Braunfärbung
Note 3: Braunfärbung
Note 4: Schwarzfärbung

### Beispiel 1

### Schwefelträger auf Basis Decen-1, Schwefelgehalt ca. 20%

Ein aus Decen-1 mit Schwefel und Schwefelwasserstoff hergestellter roher Schwefelträger wurde zunächst im Dünnschichtverdampfer bei ca. 120°C und 20 mbar von leichtflüchtigen Bestandteilen befreit, H₂S-Test: 4.

Nach Abkühlen auf 50°C wurde 1,0 Gew.-% Cumolhydroperoxidlösung (80%ig in Cumol) zugegeben und noch 1 Stunde weitergerührt, H₂S-Test: 1.

### Beispiel 2

### Schwefelträger auf Basis Decen-1, Schwefelgehalt ca. 28%

Ein aus Decen-1 mit Schwefel und Schwefelwasserstoff hergestellter roher Schwefelträger wurde zunächst im Dünnschichtverdampfer bei ca. 120°C und 20 mbar von leichtflüchtigen Bestandteilen befreit, H₂S-Test: 4.

Nach Abkühlen auf 60°C wurde 0,25 Gew.-% Cumolhydroperoxidlösung (80%ig in Cumol) zugegeben und noch 1/2 Stunde weitergerührt, H₂S-Test: 1.

### Beispiel 3

### Schwefelträger auf Basis Rüböl und Rübölfettsäuremethylester, Schwefelgehalt ca. 10%

Ein aus Rüböl und Rübölfettsäuremethylester mit Schwefel und Schwefelwasserstoff hergestellter roher Schwefelträger wurde zunächst 2 Stunden bei 100°C und 20 mbar destilliert, H₂S-Test: 4.

Nach Abkühlen auf 50°C wurde 1,0 Gew.-% Cumolhydroperoxidlösung (80%ig in Cumol) zugegeben und noch 1 Stunde weitergerührt, H₂S-Test: 1.

### Beispiel 4

### Schwefelträger auf Basis Rüböl und Rübölfettsäuremethylester und Decen-1, Schwefelgehalt ca. 15%

Ein aus Rüböl und Rübölfesttsäuremethylester und Decen-1 mit Schwefel und Schwefelwasserstoff hergestellter roher Schwefelträger wurde zunächst 2 Stunden bei 100°C und 20 mbar destilliert, H₂S-Test: 4.

Nach Abkühlen auf 50°C wurde 0,5 Gew.-% Cumolhydroperoxidlösung (80%ig in Cumol) zugegeben und noch 1 Stunde weitergerührt, H₂S-Test: 1.

### Beispiel 5

### Schwefelträger auf Basis Sonnenblumenöl und Rübölfettsäuremethylester, Schwefelgehalt ca. 15%

Ein aus Sonnenblumenöl und Rübölfettsäuremethylester mit Schwefel und Schwefelwasserstoff hergestellter roher Schwefelträger wurde ohne Desillationsschritt bei 50°C mit 0,5 Gew.-% Cumolhydroperoxidlösung (80 %ig in Cumol) versetzt und noch ½ Stunde weitergerührt, H₂S-Test: 1.

## Patentansprüche

1. Verfahren zur Verhinderung der Schwefelwasserstoff- und/oder Mercaptanemission aus geschwefelten organischen Verbindungen mit hohem Schwefelgehalt, **dadurch gekennzeichnet, daß** man die geschwefelten organischen Verbindungen mit 0,01 bis 10 Gew.-%, bezogen auf das gesamte Reaktionsgemisch, eines organischen Hydroperoxids behandelt.

2. Verfahren nach Anspruch 1, **dadurch gekennzeichnet, daß** man als organische Hydroperoxide tert.-Butylhydroperoxid, tert.-Amylhydroperoxid, Cumolhydroperoxid und/oder Diisopropylbenzolmonohydroperoxid einsetzt.

3. Verfahren nach Anspruch 1, **dadurch gekennzeichnet, daß** man Cumolhydroperoxid in Form seiner 10 bis 90 gew.-%igen Lösung in Cumol einsetzt.

## Claims

1. Process for inhibiting the emission of hydrogen sulphide and/or mercaptans from sulphurized organic compounds with a high sulphur content, **characterized in that** the sulphurized organic compounds are treated with 0.01 to 10 wt. %, with respect to the entire reaction mixture, of an organic hydroperoxide.

2. Process according to Claim 1, **characterized in that** the organic hydroperoxide used is tert.-butyl hydroperoxide, tert.-amyl hydroperoxide, cumene hydroperoxide and/or diisopropylbenzene monohydroperoxide.

3. Process according to Claim 1, **characterized in that** cumene hydroperoxide is used in the form of its 10 to 90 wt. % strength solution in cumene.

## Revendications

1. Procédé d'empêchement d'émission de sulfure d'hydrogène et/ou des mercaptans des composés organiques sulfurés à teneur élevée en soufre, **caractérisé en ce que** l'on traite les composés organiques sulfurés avec 0,01 à 10% en poids, par rapport à la totalité du mélange réactionnel, d'un hydroperoxyde organique.

2. Procédé selon la revendication 1, **caractérisé en ce que** l'on utilise comme hydroperoxydes organiques, de l'hydroperoxyde de butyle tertiaire, de l'hydroperoxyde d'amyle tertiaire, de l'hydroperoxyde de cumène et/ou du monohydroperoxyde de diisopropylbenzène.

3. Procédé selon la revendication 1, **caractérisé en ce que** l'on utilise de l'hydroperoxyde de cumène sous la forme de sa solution à 10 à 90% en poids dans du cumène.
